# EUROPEAN PATENT APPLICATION

(11) **EP 3 827 781 A1**
(43) Date of publication of application: **02.06.2021**
(21) Application number: 20209104.7
(22) Date of filing: 22.11.2020
(51) Int. Cl.: A61B 90/30, A61B 17/29, A61B 17/32, A61B 17/295, A61B 18/02

(54) **SURGICAL INSTRUMENTS WITH INTEGRATED LIGHTING SYSTEMS**

(30) Priority: 26.11.2019 US 201962940328 P
(71) Applicant: Gyrus ACMI, Inc. d/b/a Olympus Surgical Technologies America, Southborough MA 01772 (US)
(72) Inventor: MURDESHWAR, Nikhil M., Maple Grove, MN 55311 (US)
(74) Representative: Noack, Andreas

(57) **Abstract**

A surgical device comprises a shaft extending between proximal and distal portions, a surgical tool located at the distal portion, a handpiece located at the proximal portion, a lumen extending through the shaft from the proximal portion to the surgical tool, a light conductor extending into the proximal portion and at least partially through the shaft, and a light emitter connected to the light conductor to emit light from the light conductor toward the surgical tool. A method for illuminating dye while performing a laparoscopic surgical procedure comprises making an incision in a patient to form a laparoscopic port, marking target tissue for removal with a dye, inserting a laparoscopic device into the port, emitting light from a light source, passing the light through the laparoscopic device to illuminate the target tissue within the patient, and removing the target tissue with a surgical tool attached to the laparoscopic device.

## Description

### CROSS-REFERENCE TO RELATED APPLICATION

This application claims the benefit of priority to U.S. Provisional Patent Application Serial No. 62/940,328, filed on November 26, 2019, the contents of which are incorporated herein in their entirety.

### TECHNICAL FIELD

This document pertains generally, but not by way of limitation, to surgical instruments and methods that include surgical tooling that can be used for open and laparoscopic surgical procedures. More specifically, but not by way of limitation, the present application relates to systems for incorporating ancillary systems into the surgical tooling and methods for using such ancillary systems.

### BACKGROUND

Many surgical procedures involve the treatment or removal of target tissue, e.g., diseased or unwanted tissue, located inside of a patient. As such, these procedures require access to the internal anatomy of the patient via an open procedure or through a smaller opening in minimally invasive procedures. In either case, the surgeon is required to manipulate surgical instruments within tight confines of the internal anatomy to identify target tissue that is surrounded by other tissue.

Many surgical instruments exist to excise or treat target tissue that might be diseased. For example, surgical tooling, such as ablation devices, cauterizing devices and cutting forceps, can be inserted into a patient and manipulated from a handpiece that extends from the surgical tooling via a shaft. Some surgical instrumentation systems utilize lighting devices, typically from an external system, to facilitate viewing of tissue while performing a medical procedure.

Examples of surgical instruments are described in Pat. No. 6,213,995 to Steen et al.; Pat. No. US 9,851,741 to Lamser et al.; Pub. No. US 2019/0133710 A1 to Blus et al.; and Pub. No. US 2019/0282254 A1 to Fiksen et al.

### OVERVIEW

The present inventor has recognized, among other things, that problems to be solved in performing medical procedures include the inconvenience of a surgeon having to switch between utilizing multiple instruments during the procedure. In particular, laparoscopic procedures involve inserting a laparoscope tube into the patient to allow a camera to view a surgical site. Other instruments used to perform the procedure on the target tissue viewed by the camera are inserted into the tube. As such, switching between multiple instruments during any procedure, particularly laparoscopic procedures, is time consuming in switching between instruments and having to reacquire target tissue. Alternatively, making multiple incision in the patient to allow for simultaneous insertion of two separate instruments increases the time and complexity of the surgical procedure, as well as pain and recovery time for the patient.

The present inventor has also recognized that problems to be solved in performing medical procedures include the ability to properly identify target tissue for removal. For example, endometriosis is a condition in which endometrium tissue that typically lines the inside of a uterus spreads to other places in the abdomen. The condition can be particularly painful as the endometrium tissue outside the uterus continues to behave in the manner of endometrium within the uterus during the menstrual cycle by thickening, breaking-down and bleeding. Treatment for endometriosis involves removing the endometrium tissue outside the uterus. As such, it is desirable to identify the endometrium tissue such that other healthy tissue in the abdomen is not unnecessarily removed and to ensure that all of the extra-uterine endometrium tissue is identified to eliminate the endometriosis and its symptoms and the need for a follow-up procedure. Identification of endometrium tissue can be facilitated by the use of dyes whereby a patient ingests a dye that can metabolize to or otherwise be absorbed by the endometrium tissue. The dye can then be energized with light of a particular wavelength to illuminate the tissue containing the dye. However, use of dyes requires light be introduced into the surgical site, which typically requires use of an additional instrument.

The present subject matter can provide solutions to this problem and other problems, such as by providing systems incorporating light emitters into a surgical instrument in such a way that a surgical tool portion of the surgical instrument can be directly illuminated via emitted light without the need for an additional or separate tool. Methods of performing surgical procedures with such systems are also described herein. Furthermore, the light can be provided in different wavelengths to provide different energization to tissue-illuminating dyes.

In an example, a device for performing a surgical procedure can comprise a shaft extending from a proximal portion to a distal portion, a surgical tool located at the distal portion, a lumen extending through the shaft from the proximal portion to the surgical tool, a light conductor extending into the proximal portion and at least partially through the shaft, and a light emitter connected to the light conductor to emit light from the light conductor toward the surgical tool.

In another example, a cutting forceps for performing laparoscopic tissue-removal procedures can comprise a handle, a shaft extending from the handle at a proximal end to a distal end, an operational lumen extending through the shaft, a forceps disposed at the distal end and connected to the handle via a linkage extending through the operational lumen, a light conductor extending from the handle and into the shaft, and a light emitter connected to the light conductor and located proximate the distal end to illuminate tissue to be engaged by the forceps with light from the light conductor.

In an additional example, a method for illuminating dye while performing a laparoscopic surgical procedure can comprise making an incision in a patient to form a laparoscopic port, marking target tissue for removal with a dye, inserting a laparoscopic device into the port, emitting light from a light source, passing the light through the laparoscopic device to illuminate the target tissue within the patient, and removing the target tissue with a surgical tool attached to the laparoscopic device.

This overview is intended to provide an overview of subject matter of the present patent application. It is not intended to provide an exclusive or exhaustive explanation of the invention. The detailed description is included to provide further information about the present patent application.

### BRIEF DESCRIPTION OF THE DRAWINGS

FIG. 1 is a schematic illustration of a surgical instrument having an integrated lighting system according to the present disclosure.
FIG. 2 is a side view of a cutting forceps having a light conductor incorporated within a shaft and connecting a proximal light source and a distal light emitter.
FIG. 3 is a close-up view of the distal light emitter of the cutting forceps of FIG. 2.
FIG. 4 is schematic perspective view of a first example of an incorporated light conductor of the present disclosure comprising an embedded optical fiber.
FIG. 5 is a schematic cross-sectional view of the embedded light conductor of FIG. 4.
FIG. 6 is schematic perspective view of a second example of an incorporated light conductor of the present disclosure comprising a sheathed optical fiber.
FIG. 7 is a schematic cross-sectional view of the sheathed light conductor of FIG. 6.
FIG. 8 is a schematic line diagram illustrating methods for performing surgical procedures using a surgical instrument having an integrated lighting system.
FIG. 9 is a schematic diagram illustrating a laparoscopic surgical procedure to treat a patient having endometriosis.

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.

### DETAILED DESCRIPTION

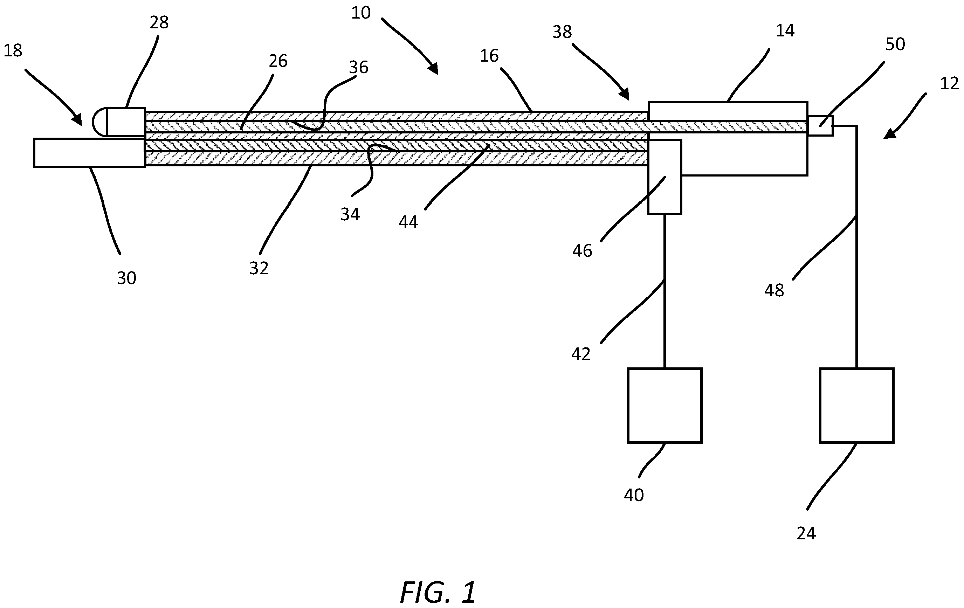

FIG. 1 is a schematic illustration of surgical instrument 10 having integrated lighting system 12 according to the present disclosure. Surgical instrument 10 can comprise handpiece or handle 14, shaft 16 and surgical device 18. Integrated lighting system 12 can comprise light source 24, light conductor 26 and light emitter 28. Surgical device 18 can comprise light emitter 28 and surgical tool 30. Shaft 16 can comprise main body 32, first lumen 34, second lumen 36, proximal end portion 38 and distal end portion 40. Surgical device 18 can further comprise power source or generator 40 that can be coupled to surgical tool 30 via cable 42 and linkage 44. Handle 14 can further comprise actuation device 46. Lighting system 12 can further comprise light source 24 connected to light conductor 26 via cable 48 and connector 50.

Integrated lighting system 12 can be configured to provide light to a working area for surgical tool 30. Integrated lighting system 12 can provide incident light directly onto tissue of a patient. Integrated lighting system 12 can additionally provide excitation energy absorbed by molecules of the tissue to activate a luminescent dye administered to the patient. Incorporation of integrated lighting system 12 can facilitate lighting of target tissue in the area surgical tool 30 to allow an operator of surgical instrument 10 to better perform a medical procedure. Furthermore, integrated lighting system 12 can reduce the length of surgical procedures by reducing or eliminating the need for a surgeon to repetitively insert and remove multiple tools from a single laparoscopic access portal or for a surgeon to manipulate and maintain multiple tools at the same time in an open procedure.

Handle 14 can comprise any device suitable for facilitating manipulation and operation of surgical instrument 10. Handle 14 can be located at the proximal end of proximal end portion 38 or another suitable location along shaft 16. In examples, handle 14 can comprise a pistol grip, a knob, a handlebar grip and the like. Actuation device 46 can be attached to handle 14 to operate linkage 44. Actuation device 46 can comprise one or more of buttons, triggers, levers, knobs, dials and the like. Linkage 44 can comprise any suitable device for allowing operation of surgical tool 30 from handle 14. Linkage 44 can be located within first lumen 34 of shaft 64 such that first lumen 34 can comprise an operational lumen of shaft 64. In examples, linkage 44 can be a mechanical linkage, an electronic linkage, an electric linkage, a fluid linkage or an acoustic linkage.

Surgical device 18 can be located at the distal end of distal end portion 440 or another suitable location along shaft 16. Surgical tool 30 can comprise a component or device for interacting with a patient, such as those configured to cut and cauterize tissue and/or produce a desired tissue effect of the patient. In examples, surgical tool 30 can comprise forceps, a cutting tool, an ablation electrode, a cryogenic needle or applicator, an ultrasonic probe tip and the like, and combinations thereof. As such, linkage 44 can comprise a mechanical linkage to actuate forceps or a cutting tool, an electrical linkage to activate an ablation electrode, an acoustic linkage, a liquid conduit (e.g., for the delivery of cryogenic argon gas) and the like, and combinations thereof. In additional examples, surgical tool 30 can comprise a device for viewing the patient, such as optical devices including endoscopes and fiberscopes.

Generator 40 can comprise a source of energy to surgical tool 30. For example, generator 40 can be configured to provide electricity for performing ablation and cauterizing functions and/or ultrasonic energy for providing cutting, coagulating, fragmenting or other types of surgical functions.

Shaft 16 can comprise an elongate member configure to allow surgical device 18 to be inserted into a patient. In examples, shaft 16 can be sized for performing laparoscopic procedures in conjunction with a laparoscope. As such, shaft 16 can be inserted into an incision in the epidermis of a patient, through a body cavity of the patient and into an organ. Thus, it is desirable for the diameter or cross-sectional shape of shaft 16 to be as small as possible to facilitate minimally invasive surgical procedures. Light conductor 26 can thus be incorporated into shaft 16 to minimize the size impact on surgical instrument 10 and without interfering with linkage 44. Shaft 16 can be rigid and formed from a metal or plastic material.

Light conductor 26 can comprise a medium for transmitting light from light source 24 to light emitter 28. Light conductor 26 can be located in second lumen 36 of shaft 64 so as to define a lighting lumen of shaft 64. Light conductor 26 can comprise a material suitable for transmitting waves of electromagnetic radiation at various wavelengths. Light conductor 26 can be coupled to light source 24 via cable 48 and connector 50. Cable 48 can comprise an extension of light conductor 26 and can be fabricated from the same material as light conductor 26. In examples, light conductor 26 and cable 48 can comprise fiber optic cables. In examples, the fiber optic cables can comprise glass and plastic fibers jacketed with one or more protective and reflective coatings. Light emitter 28 can be located at or near the distal end of light conductor 26. Light emitter 28 can be coupled to light conductor 26 by any suitable means. In examples, light emitter 28 can comprise a lens for collecting and focusing light waves from light conductor 26. Light emitter 28 can comprise a glass or plastic body of transparent material. However, in additional examples, a separate light emitter is not used and light conductor 26 can comprise an end-emitting fiber such that the distal or terminal end of light emitter 28 can comprise light emitter 28.

As mentioned, light source 24 can be coupled to light conductor 26 via cable 48. Connector 50 can comprise any suitable device for linking light conductor 26 and cable 48 such that fibers disposed therein can be adjoined in an end-to-end manner. As such, light source 24 can be located remotely from surgical instrument 10. In examples, light source 24 can comprise a stand-alone module couplable to surgical instrument 10 via cable 48. In additional examples, light source 24 can be attached directly to the exterior of handle 14 via connector 50 without using cable 48. As such, light source 24 can be removable, thereby allowing for attachment of light generators that produce different intensities or wavelengths, which, as discussed below, can allow for energization of different types of surgical dyes including fluoroscopic and near-infrared dyes. In additional examples, light source 24 can be incorporated into handle 14 such that connector 50 is not used. In additional examples, light source 24 can be incorporated into generator 40 and cable 48 and cable 42 can be included in a common cable bundle.

As mentioned above and discussed in further detail below, light from light source 24 can be transmitted through shaft 16 using light conductor 26 to illuminate work space of surgical device 18 with or without the aid of a separate light emitter device, such as light emitter 28. Thus, light can be delivered without increasing the size of an incision or the number of incisions in a patient required to deliver both the light and surgical device 18. This can reduce the time of surgical procedures, the number of hands needed to perform a surgical procedure, and the pain and healing time of the patient. Furthermore, the light can be used to illuminate dyes administered to a patient to enhance the contrast of tissue in the patient. As mentioned, removable light generators can facilitate production of light at different wavelengths.

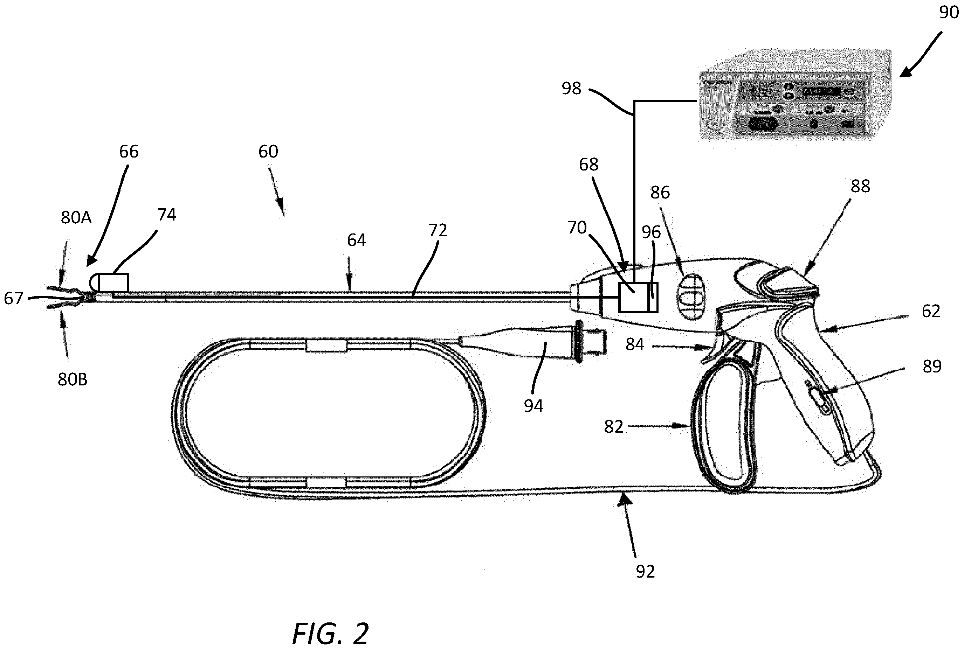

FIG. 2 is a side view of cutting forceps 60, which can comprise a specific example of surgical instrument 10 of FIG. 1. Cutting forceps 60 can comprise handpiece 62, shaft 64, jaw assembly 66, blade 67 and lighting system 68. Lighting system 68 can comprise light generator 70, light conductor 76 and light emitter 78. Jaw assembly 66 can comprise first jaw element 80A and second jaw element 80B. Handpiece 62 can comprise one or more user inputs or controls, such as lever 82, trigger 84, wheel 86, button 88 and switch 89. Cutting forceps 60 can be coupled to generator 90 via cable 92 and connector 94.

Cutting forceps 60 can be configured as described in Pat. No. US 9,851,741 to Lamser et al. modified to include lighting system 68. US 9,851,741 to Lamser et al. is hereby incorporated by this reference in its entirety.

Cutting forceps 60 can be configured to provide electro-cautery functionality to jaw assembly 66. Blade 67 can be actuated relative to jaw assembly 66 to resect tissue held by jaw elements 80A and 80B. Lighting system 68 can be configured to emit light to illuminate tissue and energize fluorescent dye within the tissue, such as tissue being held between jaw elements 80A and 80B and tissue in the vicinity of jaw assembly 66.

Shaft 64 can be inserted into a laparoscope such that jaw assembly 66 can be located within a patient in the vicinity of target tissue that is to be removed from the patient. Wheel 86 can be rotated to rotate shaft 64 relative to handpiece 62. As such, a surgeon can position jaw elements 80A and 80B about the main longitudinal axis of shaft 64.

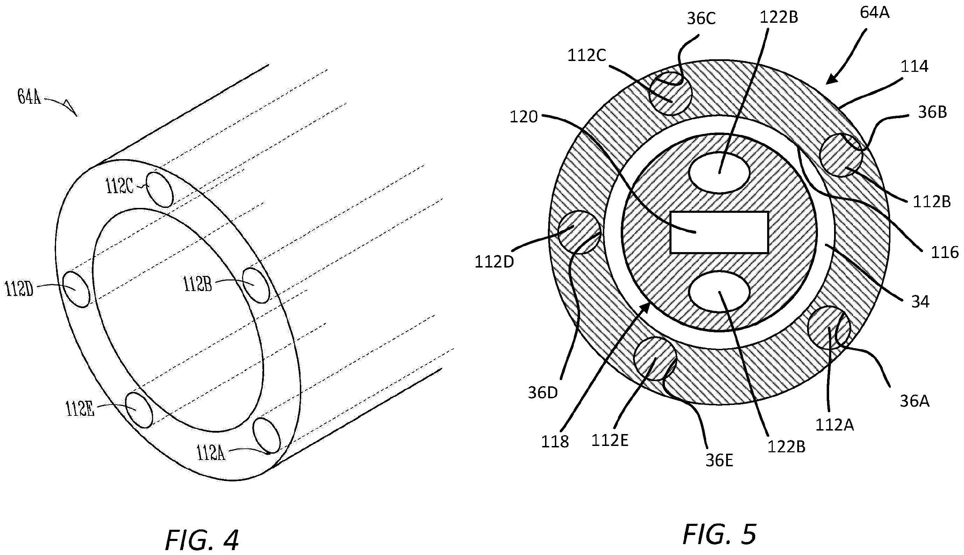

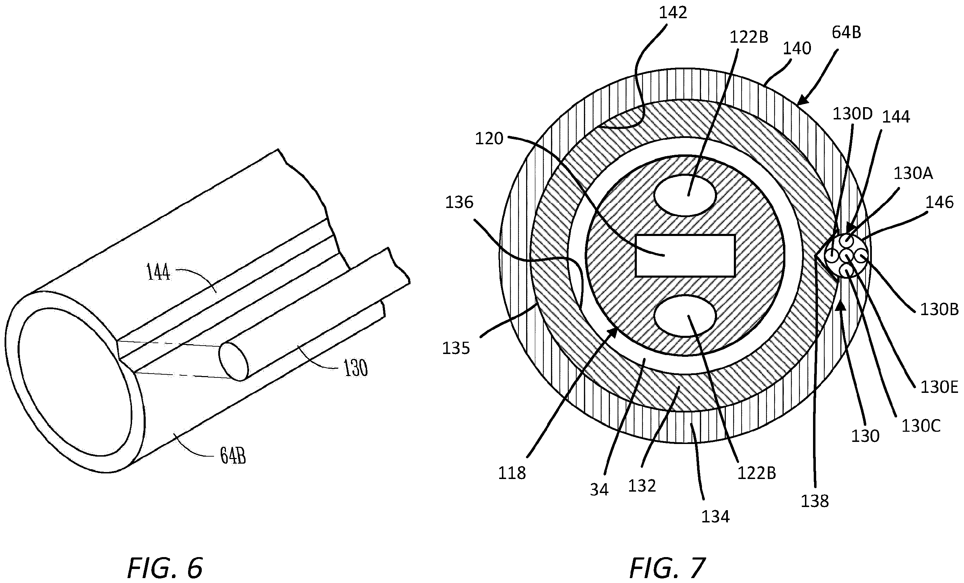

Lever 82 can be depressed, that is, pivoted toward handpiece 62, to cause jaw elements 80A and 80B to be brought together to, for example, clamp down on target tissue. In particular, lever 82 can be configured to move a mount for shaft 64 disposed inside of handpiece 62 forward, or distally, while jaw elements 80A and 80B are axially anchored to handpiece 62, such as via engagement with a mounting component inside handpiece 62. In other words, jaw elements 80A and 80B can be anchored within handpiece 62 via support rods (122A and 122B of FIGS. 5 and 7) that extend through shaft 64. Thus, movement of shaft 64 over jaw elements 80A and 80B can force jaw elements toward each other. Switch 89 can be actuated to immobilize jaw elements 80A and 80B in a latched position.

Trigger 84 can be depressed, that is, pushed toward handpiece 62, to cause blade 67 to extend from shaft 64 into the space between jaw elements 80A and 80B to cut the target tissue. As such, the target tissue can be separated from the patient and held between jaw elements 80A and 80B whereby the surgeon can manipulate cutting forceps to remove the resected target tissue from the patient, such as by removing shaft 62 from the patient or positioning the resected target tissue near a suction device.

Additionally, before or after actuation of trigger 84, button 88 can be depressed, that is, slid along handpiece 62, to cause energy from generator 90 to be delivered to jaw elements 80A and 80B to heat and cauterize the tissue adjacent the target tissue to be removed to limit or prevent bleeding, etc. Furthermore, generator 90 can be configured to provide mechanical energy to jaw elements 80A and 80B in the form of ultrasonic energy waves. Such mechanical energy can be used to cut tissue.

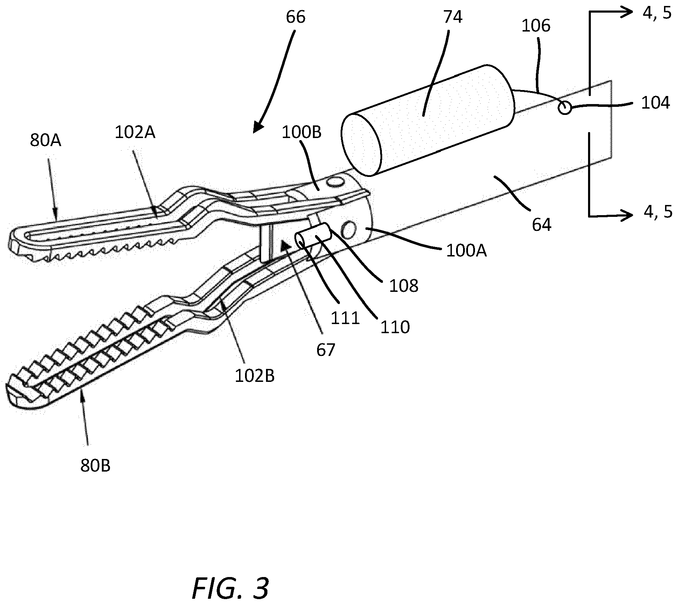

In order to allow the surgeon to better see or visualize the target tissue, lighting system 68 can be configured to emit light in the direction of jaw elements 80A and 80B. Light from light generator 70 can be transmitted to the distal end portion of shaft 64 via light conductor 72. Light conductor 72 is incorporated into shaft 64 to protect light conductor 72 and minimize the size of shaft 64. In examples, light generator 70 can be incorporated into or placed inside of handpiece 62. In examples, light generator 70 can be placed along shaft 64 distally of handpiece 62 and proximally of light emitter 74. For example, light generator 70 can be positioned along shaft 64 distally of handpiece 62 along a portion of shaft 64 not intended to be positioned within a patient. In additional examples, light generator 70 can be positioned along a portion of shaft 64 intended to be inserted within a patient, but proximally of light emitter 74 at the distal most portion of shaft 64 to minimize the size of the working end of cutting forceps 60 and facilitate access to tight confines within the anatomy. Light generator 70 can include switch 96 to control generation of light from light generator or transmission of light into light conductor 72. Light conductor 72 can extend from light generator 70 to light emitter 74. Light generator 70 can additionally be connected to generator 90 via cable 98. Generator 98 can supply power to light generator 70 via cable 98. Light emitter 74 can be mounted on shaft 64 in order to provide illumination of jaw assembly 66 and tissue that is distal of jaw assembly 66, as discussed in greater detail with reference to FIG. 3.

FIG. 3 is a close-up view of light emitter 74 of cutting forceps 60 of FIG. 2 located at a distal end of shaft 64. Shaft 64 can further comprise end caps 100A and 100B and jaw elements 80A and 80B can further comprise slots 102A and 102B, respectively. As illustrated, cutting forceps 60 can be provided with light emitter 74 located adjacent shaft 64. However, additional light emitters can be included, or a specific light-emitting device used in addition to light conductor 72 can be omitted, and light can be left to freely be discharged from the ends of optical fibers, as shown by fiber cable 110 in FIG. 3.

Shaft 64 can include one or more end caps, such as end caps 100A and 100B, to protect the distal end of shaft 64 and to guide jaw elements 80A and 80B. Two other end caps similar to end caps 100A and 100B can be provided, though not visible in FIG. 3, such that another end cap is between jaw elements 80A and 80B and another jaw element is within slot 102B of jaw element 80B. Jaw elements 80A and 80B can protrude from the distal end of shaft 64 from within end caps 100A and 100B. Jaw elements 80A and 80B can include slots 102A and 102B, respectively, to allow blade 67 to extend into jaw elements 80A and 80B when jaw elements 80A and 80B care closed and blade 67 is extended.

Shaft 64 can additionally include bore 104 to allow fiber cable 106 to exit shaft 64. Fiber cable 106 can comprise one or more fiber cables comprising light conductor 72 (FIG. 2). Fiber cable 106 can be connected to light emitter 74. Light emitter 74 can be positioned in the distal end region of shaft 64 to project light toward jaw elements 80A and 80B. Light from light emitter 74 can be projected beyond jaw elements 80A and 80B to illuminate tissue in front of or distal to jaw elements 80A and 80B to allow a surgeon to see target tissue locations where cutting forceps 60 can be moved to treat tissue. Bore 104 can comprise any opening in shaft 64 to allow one or more incorporated light conductors, such as those of FIGS. 4 - 7, to pass therethrough. Bore 104 can be sealed in any suitable manner, such as with an epoxy or an elastomeric O-ring. FIG. 3 illustrates a single light emitter 74 attached to shaft 64. However, one or more light emitters 74 can be provided around the circumference of shaft 64. Each of the plurality of light emitters 74 can be provided with light conductors from bore 104 or from a different bore dedicated to each light emitter.

Additionally, as mentioned above, shaft 64 can be provided with distal bore 108 to allow a distal, light-emitting end of fiber cable 110 to exit shaft 64. Fiber cable 110 can include end surface 111 that can comprise a facet of fiber cable 110, such as can be formed with a cut. End surface 111 can be directionally cut to emit light in a desired orientation. As shown, end surface 111 can be oriented perpendicular to the central axis of shaft 64. However, end surface 111 can be oriented to emit light toward the central axis of shaft 64 so as to direct light toward jaw elements 80A and 80B or oriented to emit light away from the central axis of shaft 64 so as to direct light toward surrounding tissue. Fiber cable 110 can comprise one or more fiber cable comprising light conductor 72 (FIG. 2). Distal bore 108 can be located in end cap 100A or an un-capped end of shaft 64. Bore 110 can be sealed in any suitable manner, such as with an epoxy or an elastomeric O-ring.

The size of light emitter 74 and fiber cable 110 are exaggerated in FIG. 3 for visualization purposes. However, it is advantageous for the various embodiments of lighting system 68 to minimize any size increases to shaft 64 to allow cutting forceps to remain functional with laparoscope tubes. As such, the size of light emitter 74 and fiber cable 110 and the number of light emitters 74 and fiber cables 110 can be selected to balance lighting needs and size constraints.

FIG. 4 is schematic perspective view of a first example of an incorporated light conductor of the present disclosure comprising optical fibers 112A - 112E embedded into shaft 64A. FIG. 5 is a schematic cross-sectional view of shaft 64A of FIG. 4 including embedded light conductors 112A - 112E. FIGS. 4 and 5 are discussed concurrently. Shaft 64A can comprise an embodiment of shaft 64 of FIG. 1.

Shaft 64A can comprise a tubular element having outer wall 114 and inner wall 116 that defines first lumen 34 (FIG. 1). Optical fibers 112A - 112B can be embedded into second lumens 36A - 36E. Second lumens 36A - 36D can comprise embodiments of second lumen 36 of FIG. 1. Shaft 64A can comprise internal body 118. Internal body 118 can be sized to fit within lumen 34. The outer surface of internal body 118 can be configured to engage inner wall 116 of shaft 64A but is illustrated as being spaced therefrom as can be the case in other examples. Internal body 118 can comprise a central passage for receiving drive rod 120 for cutting blade 67 (FIG. 3), a first peripheral passage for receiving first support rod 122A for first jaw element 80A, and a second peripheral passage for receiving second support rod 122B for second jaw element 80B. Drive rod 120 can be free to move within internal body 118 as button 84 (FIG. 2) is actuated. Internal body 118 can be configured to move along first support rod 122A and second support rod 122B as trigger 82 is actuated in examples where internal body 118 is attached to shaft 64A. However, internal body 118 can be configured to be stationary relative to first support rod 122A and second support rod 122B as trigger 82 is actuated in examples where shaft 64A is configured to slide over internal body 118.

Optical fibers 112A - 112E can be embedded within shaft 64A between outer wall 114 and inner wall 116, within second lumens 36A - 36D, respectively. As such, optical fibers 112A - 112E can be fully encased and protected by material of shaft 64A. In the illustrated example, shaft 64A includes five embedded optical fibers 112A - 112E. However, shaft 64A can be configured to include additional or fewer fibers. Likewise, in the illustrated example, optical fibers 112A - 112E are shown evenly spaced around the circumference of shaft 64A. However, optical fibers can be unevenly distributed around the circumference of shaft 64A or concentrated on one segment of shaft 64A to provide directional lighting or varying levels of brightness. Additionally, FIG. 5 illustrates optical fibers 112A -112E as comprising a single fiber. However, in other examples optical fibers 112A - 112E can comprise a bundle of fibers. Optical fibers 112A - 112E can be configured to transmit light signals from an end proximate a light source to a light emitter. Optical fibers 112A - 112E can receive light waves and the light waves can be propagated down the core of the optical fibers, such as by bouncing off the sidewall of the optical fibers. The bouncing of the light waves can be enhanced by reflective or mirrored coating applied to the outer sidewall. As such, the light waves are not absorbed by the core or sidewall and can travel between the light source and the light emitter without being diminished.

Shaft 64A can be manufactured to include optical fibers 112A - 112E embedded therein. In examples, shaft 64A can extruded with optical fibers 112A - 112E or fibers 112A - 112E can be added into shaft 64A in a layered build-up of coatings. The configuration of shaft 64A can be advantageous for embodiments of shaft 64A fabricated from plastic materials.

FIG. 6 is schematic perspective view of a second example of an incorporated light conductor of the present disclosure comprising optical fiber bundle 130 sheathed within shaft 64B. FIG. 7 is a schematic cross-sectional view of shaft 64B of FIG. 6 including sheathed optical fiber bundle 130. FIGS. 6 and 7 are discussed concurrently. Shaft 64B can comprise an embodiment of shaft 64 of FIG. 1.

Shaft 64B can comprise a tubular element 132 and sheath 134. Tubular element 132 can comprise outer wall 135 and inner wall 136 that defines first lumen 34 (FIG. 1). Outer wall 135 can comprise groove 138 into which optical fiber bundle 130 can be disposed. Optical fiber bundle 130 can comprise strands 130A - 130E. Sheath 134 can comprise annular body having outer wall 140 and inner wall 142. Shaft 64B can comprise lumen 144, which can comprise an embodiment of second lumen 36 (FIG. 1). Lumen 144 can comprise groove 138 and an opposing portion of sheath 134, such as indentation 146.

Shaft 64B can comprise internal body 118, which can be configured the same as is described with reference to FIGS. 4 and 5.

Groove 138 can be formed in outer wall 135 of tubular element 132 during manufacturing. Groove 138 can have a "V" shape, as illustrated, but can have other shapes in other embodiments. Groove 138 can be formed by any suitable means including during the forming of tubular element 132 itself or thereafter with a subtractive manufacturing technique, such as milling or cutting. Optical fiber bundle 130 can be laid in groove 138 and can be held in place using an adhesive. Sheath 134 can be placed over tubular element 132 and optical fiber bundle 130 using any suitable method. Sheath 134 can have a sufficient thickness such that outer wall 140 will by smooth (e.g., free of circumferential discontinuities). For example, sheath 134 can be sufficiently thick such that indentation 146 is not noticeable in outer wall 140.

For example, sheath 134 can comprise a plastic tube that can be shrink-wrapped around tubular element 132. The configuration of shaft 64B can be advantageous for embodiments of shaft 64B fabricated from metallic materials.

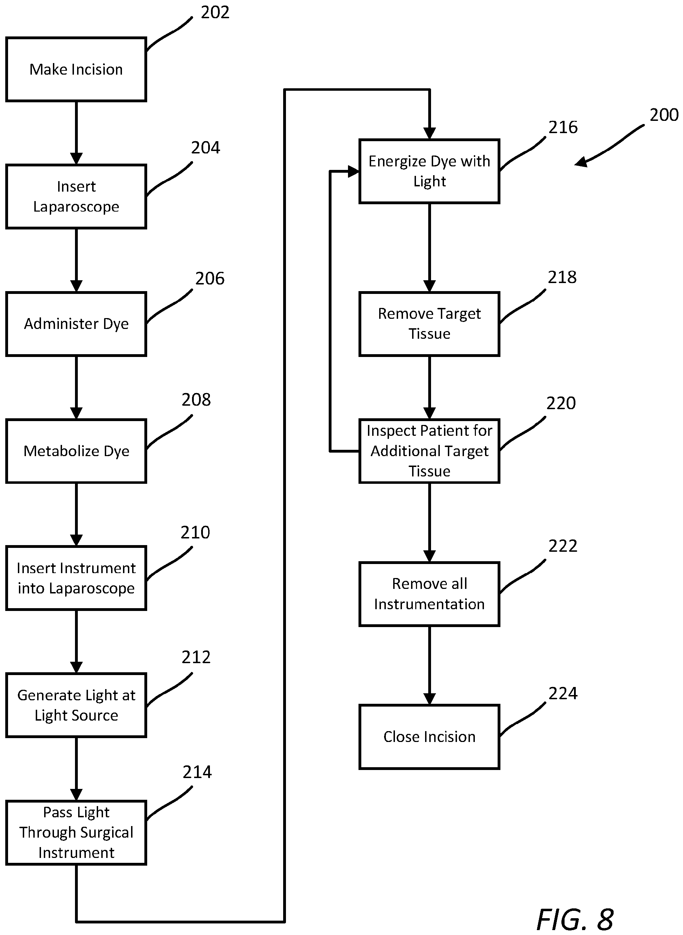

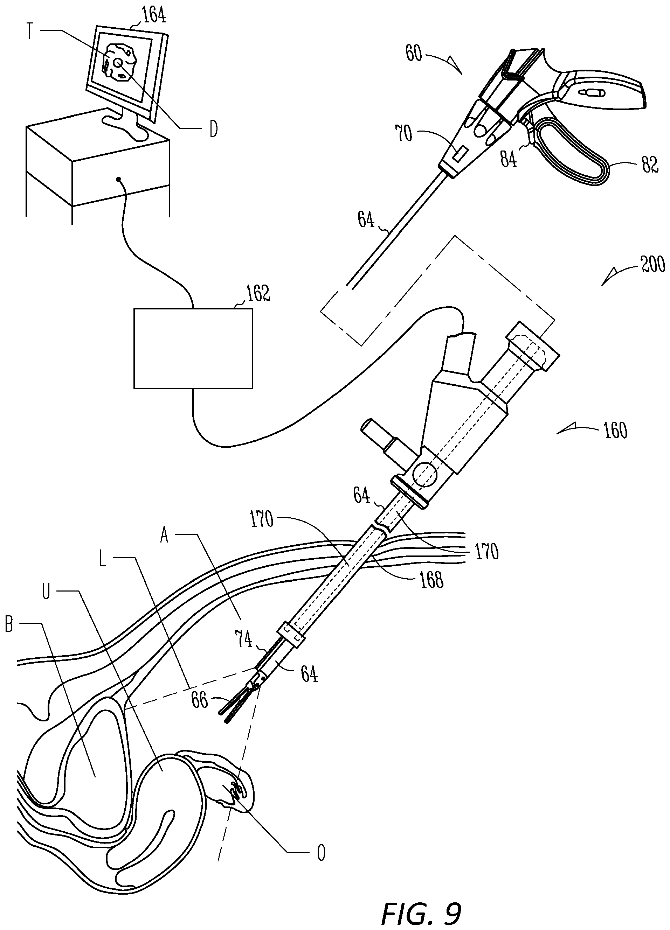

FIG. 8 is a line diagram illustrating method 200 for performing a surgical procedure according the present disclosure. FIG. 9 is a schematic diagram of a laparoscopic surgical procedure being performed according to method 200 of FIG. 8. Elements of FIG. 9 are not drawn to scale for illustrative purposes. FIGS. 8 and 9 are discussed concurrently.

The surgical procedure can comprise an open procedure or a laparoscopic procedure. FIG. 9 illustrates a laparoscopic procedure being performed with cutting forceps 60 of FIG. 2. The surgical procedure can be performed to remove or otherwise abate target tissue that is diseased or invasive. FIG. 9 illustrates a surgical procedure being performed to remove endometrium tissue from the cavity of abdomen A that has grown outside of uterus U. However, the instruments and methods of the present application can be used to perform other procedures, such as breast cancer surgeries to remove sentinel lymph node (SLN) tissue. In another example, the instruments and methods of the present disclosure can be used to perform anastomosis procedures where knowing the degree of tissue perfusion is important. The surgical procedure can be performed in an operating room in a hospital or out-patient facility. FIG. 9 illustrates an operating room environment where laparoscope 160 is coupled to camera 162 and display 164. The patient can be appropriately anesthetized.

At step 202, incision 168 can be made in abdomen A of the patient. Incision 168 can be an incision having a sufficient length to form a portal for performing an open procedure. Incision 168 can also be a minimally invasive incision, such as one configured to receive laparoscope 160 as shown in FIG. 9.

At step 204, laparoscope 160 can be inserted into incision 168. Laparoscope 160 can be coupled to camera 162 for viewing tissue within abdomen A of the patient internal to incision 168. Laparoscope 160 can include passage 170 that extends through incision 168 to allow access to internal tissue of the patient from outside the patient.

At step 206, a dye can be administered to a patient. The dye can be ingested or administered intravenously. The dye can comprise any type of dye used for surgical procedures. For example, blue dyes (methylene blue) can be used in cancer surgeries. Also, fluorescent dyes, such as indocyanine green (ICG), can be used in endometriosis surgeries.

At step 208, the dye can be metabolized or otherwise absorbed into the tissue of the patient, including by tissue that is to be targeted by the surgeon for abatement. The tissue of uterus U, bladder B, ovaries O and other locations and organs, such as fallopian tubes and the rectum, can metabolize the dye. Step 204 can alternatively be performed pre-operatively or intraoperatively before step 202.

At step 210, a surgical instrument having a surgical tool, such as surgical instrument 10 having surgical device 18 of FIG. 1, can be inserted into incision 168. For example, cutting forceps 60 can be inserted into passage 170 of laparoscope 160. Specifically, shaft 64 of laparoscope 160 can be inserted through passage 170 such that jaw assembly 66 protrudes from passage 170 and is located inside abdomen A. Likewise, light emitter 74 can be positioned within abdomen A.

At step 212, light can be generated by a light source to be emitted into the cavity abdomen A. The light source can be attached to the surgical instrument. The light can be white light to visually aid viewing of tissue. The light can additionally be light of a wavelength sufficient to energize, e.g., fluoresce, the dye, such as near-infrared (NIR) light. NIR light can be used to energize indocyanine green in endometriosis surgeries. NIR light is typically located in the near-infrared region of the electromagnetic spectrum, from approximately 780 nm to approximately 2500 nm. The indocyanine green dye can concentrate in vascular rich areas, such as in endometrium tissue.

At step 214, the light from the light generator can be passed through the surgical instrument including the surgical device. For example, the light can be emitted from light generator 70, pass through light conductor 72 (FIG. 2) and into light emitter 74.

At step 216, light can be emitted from a light emitter of the surgical tool inserted into abdomen A through incision 168. For example, light L from light emitter 74 integrated into shaft 64 can be directed toward target tissue outside of uterus U. Light L can energize dye metabolized into tissue of abdomen A. In particular, damaged, diseased or otherwise undesirable tissue can metabolize the dye in such a way that light L will allow that tissue to be more readily distinguished from neighboring dye. Energized dye can be viewed by camera 162 through laparoscope 160. Video from camera 162 can be viewed by an eyepiece attached to laparoscope 160 or at display 164. For example, tissue T can include dye D, indicating target tissue to be removed can be shown in display 164, which can comprise a video monitor.

At step 218, target tissue identified by the light can be treated with the surgical tool of the surgical instrument. For example, light L emitted from shaft 64 can pass around jaw assembly 66 to illuminate tissue on ovary O. Lever 82 of forceps 60 can be operated to grasp the target tissue and trigger 84 of forceps 60 can be operated to cut the target tissue and separate the target tissue from abdomen A. For example, monopolar energy or bipolar energy from generator 90 (FIG. 2) can be directed through support rods 122A and 122B to cut the target tissue.

At step 220, the patient can be inspected for additional target tissue. In particular, the surgical instrument can be moved around within abdomen A to move the light to illuminate different tissue. For example, laparoscope 160 can be tilted in incision 168, shaft 64 can be inserted further into or retracted further out of abdomen A, and shaft 64 can be rotated using wheel 86 to change where light L is directed.

Steps 216 and 218 can be repeated as necessary to ensure that all target tissue has been removed from the patient. After it is determined that no additional tissue is to be removed, the patient can be prepared to end the procedure and close incision 168.

At step 222, all instrumentation can be removed from the patient. For example, cutting forceps 60 can be removed from laparoscope 160 and laparoscope 160 can be removed from incision 168.

At step 224, the incision can be closed. For example, incision 168 can be sutured or closed using any suitable means.

The benefits of the systems and methods of the present disclosure can be in the form of, for example, 1) combining light emitting capabilities at the tool-end of a surgical instrument, 2) eliminating the need to rotate between lighting instruments and tissue-interacting instruments in a single laparoscope tube, 3) facilitating identification of all target tissue in a single medical procedure, 4) reducing times to perform surgical procedures, and 5) reducing the need for post-surgery pathology testing.

### Various Notes and Examples

Example 1 is a device for performing a surgical procedure, the device comprising: a shaft extending from a proximal portion to a distal portion; a surgical tool located at the distal portion; a lumen extending through the shaft from the proximal portion to the surgical tool; a light conductor extending into the proximal portion and at least partially through the shaft; and a light emitter connected to the light conductor to emit light from the light conductor toward the surgical tool.
In Example 2, the subject matter of Example 1 optionally includes wherein the surgical tool comprises an instrument for treating tissue.
In Example 3, the subject matter of Example 2 optionally includes wherein the surgical tool comprises an ablator selected from the group consisting of a thermal-ablator, a cryo-ablator, an electro-ablator and an ultrasonic-ablator.
In Example 4, the subject matter of any one or more of Examples 1-3 optionally include wherein the surgical tool comprises an optical device for viewing tissue.
In Example 5, the subject matter of any one or more of Examples 1-4 optionally include a light generator coupled to the light conductor.
In Example 6, the subject matter of Example 5 optionally includes a handpiece located at the proximal portion; and a switch located on the handpiece to selectively operate the light generator, wherein the light generator is within the handpiece or between the handpiece and the distal portion.
In Example 7, the subject matter of any one or more of Examples 5-6 optionally include wherein the light generator comprises a near-infrared light source.
In Example 8, the subject matter of any one or more of Examples 1-7 optionally include wherein: the light conductor comprises an optical fiber; and the light emitter comprises an end surface of the optical fiber.
In Example 9, the subject matter of any one or more of Examples 1-8 optionally include wherein the light conductor is outside of the lumen.
In Example 10, the subject matter of any one or more of Examples 1-9 optionally include wherein the shaft comprises: a tubular body defining the lumen, the tubular body comprising: an inner wall; an outer wall; and a channel extending along the tubular body to receive the light conductor.
In Example 11, the subject matter of Example 10 optionally includes wherein the channel comprises a groove in the outer wall.
In Example 12, the subject matter of Example 11 optionally includes a sheath at least partially surrounding the tubular body to secure the light conductor in the groove.
In Example 13, the subject matter of any one or more of Examples 10-12 optionally include wherein the channel comprises a passageway between the inner wall and the outer wall.
In Example 14, the subject matter of any one or more of Examples 10-13 optionally include multiple channels extending along the tubular body; and a light conductor disposed in respective ones of the multiple channels.
Example 15 is a cutting forceps for performing laparoscopic tissue-removal procedures, the cutting forceps comprising: a handle; a shaft extending from the handle at a proximal end to a distal end; an operational lumen extending through the shaft; a forceps disposed at the distal end, the forceps connected to the handle via a linkage extending through the operational lumen; a light conductor extending from the handle and into the shaft; and a light emitter connected to the light conductor and located proximate the distal end to illuminate tissue to be engaged by the forceps with light from the light conductor.
In Example 16, the subject matter of Example 15 optionally includes wherein the shaft comprises: a tubular metal body defining the operational lumen, the tubular metal body comprising: an inner wall; an outer wall; and a groove in the outer wall to receive the light conductor; and a sheath at least partially covering the tubular metal body to hold the light conductor in the groove.
In Example 17, the subject matter of any one or more of Examples 15-16 optionally include wherein the shaft comprises: a tubular polymer body defining the operational lumen, the tubular polymer body comprising: an inner wall; an outer wall; and a passageway between the inner wall and the outer wall to receive the light conductor.
In Example 18, the subject matter of any one or more of Examples 15-17 optionally include a light generator coupled to the light conductor, the light generator comprising a near-infrared light source; wherein the light conductor comprises an optical fiber.
In Example, 19, the subject matter of any one or more of Examples 15-18 optionally include a light generator coupled to a proximal end of the light conductor, the light generator mounted to the handle or mounted to the shat proximally of the light emitter; and a switch located on the handle to selectively operate the light generator.
In Example 20, the subject matter of any one or more of Examples 15-19 optionally include wherein the forceps comprise: first and second jaws coupled to the shaft, the first and second jaws being pivotable relative to each other; and a cutting blade extendable from the distal end of the shaft between the first and second jaws; wherein the light source is positioned to illuminate the first and second jaws and the cutting blade.
Example 21 is a method for illuminating while performing a laparoscopic surgical procedure, the method comprising: making an incision in a patient to form a laparoscopic port; marking target tissue for removal with a dye; inserting a laparoscopic device into the port; emitting light from a light source; passing the light through the laparoscopic device to illuminate the target tissue within the patient; and removing the target tissue with a surgical tool attached to the laparoscopic device.
In Example 22, the subject matter of Example 21 optionally includes emitting the light from a light emitter located at a distal portion of the laparoscopic device.
In Example 23, the subject matter of any one or more of Examples 21-22 optionally include wherein emitting the light from the light source comprises generating the light with a light generator attached to the laparoscopic device.
In Example 24, the subject matter of any one or more of Examples 21-23 optionally include wherein passing the light through the laparoscopic device comprises passing the light through an optical fiber within a shaft of the laparoscopic device.
In Example 25, the subject matter of any one or more of Examples 21-24 optionally include wherein passing the light through the laparoscopic device to illuminate the target tissue within the patient comprises illuminating dye with near-infrared light.
In Example 26, the subject matter of Example 25 optionally includes wherein marking target tissue for removal with the dye comprises intravenously administering indocyanine green dye to the patient.
In Example 27, the subject matter of any one or more of Examples 21-26 optionally include wherein removing the target tissue with a surgical tool attached to the laparoscopic device comprises removing endometrial tissue.
In Example 28, the subject matter of any one or more of Examples 21-27 optionally include wherein removing the target tissue with a surgical tool attached to the laparoscopic device comprises cutting the target tissue with forceps.
In Example 29, the subject matter of any one or more of Examples 21-28 optionally include wherein removing the target tissue with a surgical tool attached to the laparoscopic device comprises ablating the target tissue with an electrode, freezing the target tissue with a cryogenic applicator or cutting the target tissue with ultrasonic energy.
In Example 30, the subject matter of any one or more of Examples 21-29 optionally include wherein emitting the light from the light source comprises generating the light with a light generator connected to a generator for the laparoscopic device.
Example 31 is a method for illuminating while performing an open or laparoscopic surgical procedure, the method comprising: injecting dye into a patient to cause a visual delineation between background tissue and foreground tissue with the dye; illuminating the dye to visualize delineated tissue within target tissue; and performing a surgical procedure to introduce a tissue effect at the target tissue.
In Example 32, the subject matter of Example 31 optionally includes wherein illuminating the dye to visualize delineated tissue within the target tissue and performing a surgical procedure to introduce a tissue effect at the target tissue are preformed simultaneously.
In Example 33, the subject matter of Example 32 optionally includes wherein illuminating the dye to visualize delineated tissue within the target tissue and performing a surgical procedure to introduce a tissue effect at the target tissue are preformed using a single surgical instrument.
In Example 34, the subject matter of Example 33 optionally includes wherein the target tissue comprises endometrium tissue.
In Example 35, the subject matter of Example 34 optionally includes wherein the tissue effect comprises removing the endometrium tissue with cutting forceps.

Each of these non-limiting examples can stand on its own, or can be combined in various permutations or combinations with one or more of the other examples.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventor also contemplates examples in which only those elements shown or described are provided. Moreover, the present inventor also contemplates examples using any combination or permutation of those elements shown or described (or one or more aspects thereof), either with respect to a particular example (or one or more aspects thereof), or with respect to other examples (or one or more aspects thereof) shown or described herein.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their obj ects.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

## Claims

1. A device for performing a surgical procedure, the device comprising:
a shaft extending from a proximal portion to a distal portion;
a surgical tool located at the distal portion;
a lumen extending through the shaft from the proximal portion to the surgical tool;
a light conductor extending into the proximal portion and at least partially through the shaft; and
a light emitter connected to the light conductor to emit light from the light conductor toward the surgical tool.

2. The device of claim 1, wherein the surgical tool comprises an instrument for treating tissue.

3. The device of claim 2, wherein the surgical tool comprises an ablator selected from the group consisting of a thermal-ablator, a cryo-ablator, an electro-ablator and an ultrasonic-ablator.

4. The device of claim 1, wherein the surgical tool comprises an optical device for viewing tissue.

5. The device of claim 1, further comprising a light generator coupled to the light conductor.

6. The device of claim 5, further comprising:
a handpiece located at the proximal portion; and
a switch located on the handpiece to selectively operate the light generator, wherein the light generator is within the handpiece or between the handpiece and the distal portion.

7. The device of claim 5, wherein the light generator comprises a near-infrared light source.

8. The device of claim 1, wherein:
the light conductor comprises an optical fiber; and
the light emitter comprises an end surface of the optical fiber.

9. The device of claim 1, wherein the light conductor is outside of the lumen.

10. The device of claim 1, wherein the shaft comprises:
a tubular body defining the lumen, the tubular body comprising:
an inner wall;
an outer wall; and
a channel extending along the tubular body to receive the light conductor.

11. The device of claim 10, wherein the channel comprises a groove in the outer wall.

12. The device of claim 11, further comprising a sheath at least partially surrounding the tubular body to secure the light conductor in the groove.

13. The device of claim 10, wherein the channel comprises a passageway between the inner wall and the outer wall.

14. The device of claim 10, further comprising:
multiple channels extending along the tubular body; and
a light conductor disposed in respective ones of the multiple channels.

15. A cutting forceps for performing laparoscopic tissue-removal procedures, the cutting forceps comprising:
a handle;
a shaft extending from the handle at a proximal end to a distal end;
an operational lumen extending through the shaft;
a forceps disposed at the distal end, the forceps connected to the handle via a linkage extending through the operational lumen;
a light conductor extending from the handle and into the shaft; and
a light emitter connected to the light conductor and located proximate the distal end to illuminate tissue to be engaged by the forceps with light from the light conductor.

16. The cutting forceps of claim 15, wherein the shaft comprises:
a tubular metal body defining the operational lumen, the tubular metal body comprising:
an inner wall;
an outer wall; and
a groove in the outer wall to receive the light conductor; and
a sheath at least partially covering the tubular metal body to hold the light conductor in the groove.

17. The cutting forceps of claim 15, wherein the shaft comprises:
a tubular polymer body defining the operational lumen, the tubular polymer body comprising:
an inner wall;
an outer wall; and
a passageway between the inner wall and the outer wall to receive the light conductor.

18. The cutting forceps of claim 15, further comprising:
a light generator coupled to the light conductor, the light generator comprising a near-infrared light source;
wherein the light conductor comprises an optical fiber.

19. The cutting forceps of claim 15, further comprising:
a light generator coupled to a proximal end of the light conductor, the light generator mounted to the handle or mounted to the shat proximally of the light emitter; and
a switch located on the handle to selectively operate the light generator.

20. The cutting forceps of claim 15, wherein the forceps comprise:
first and second jaws coupled to the shaft, the first and second jaws being pivotable relative to each other; and
a cutting blade extendable from the distal end of the shaft between the first and second jaws;
wherein the light source is positioned to illuminate the first and second jaws and the cutting blade.
